# EUROPÄISCHE PATENTANMELDUNG

(11) **EP 2 201 899 A1**
(43) Veröffentlichungstag der Anmeldung: **30.06.2010**
(21) Anmeldenummer: 08172680.4
(22) Anmeldetag: 23.12.2008
(51) Int. Cl.: A61B 17/02, A61F 9/007, A61B 17/66, A61B 19/00, A61B 17/80

(54) **Instrument zu Operation einer Augenhöhlenbodenfraktur, Satz aus einer Mehrzahl solcher Instrumente und Verfahren zur Herstellung eines solchen Instruments**

(71) Anmelder: Hibrand Anstalt, 9491 Ruggell (LI)
(72) Erfinder: Halbeisen, Otmar, 9442 Berneck (CH)
(74) Vertreter: Winter, Brandl, Fürniss, Hübner, Röss, Kaiser, Polte - Partnerschaft

(57) **Zusammenfassung**

Ein Instrument (34) zur Operation einer Augenhöhlenbodenfraktur ist gekennzeichnet durch einen löffelförmigen Endabschnitt (36), dessen Form sich an eine Form eines medialen Augenhöhfenbodens anschmiegt, und einen an einem Ende mit dem löffelartigen Endabschnitt verbundenen Griffabschnitt (38).

## Beschreibung

Die vorliegende Erfindung betrifft ein Instrument zur Operation einer Augenhöhlenbodenfraktur gemäß Anspruch 1, d. h. ein Instrument, das bei einer solchen Operation einsetzbar ist, einen Satz aus einer Mehrzahl solcher Instrumente sowie ein Verfahren zur Herstellung eines solchen Instruments.

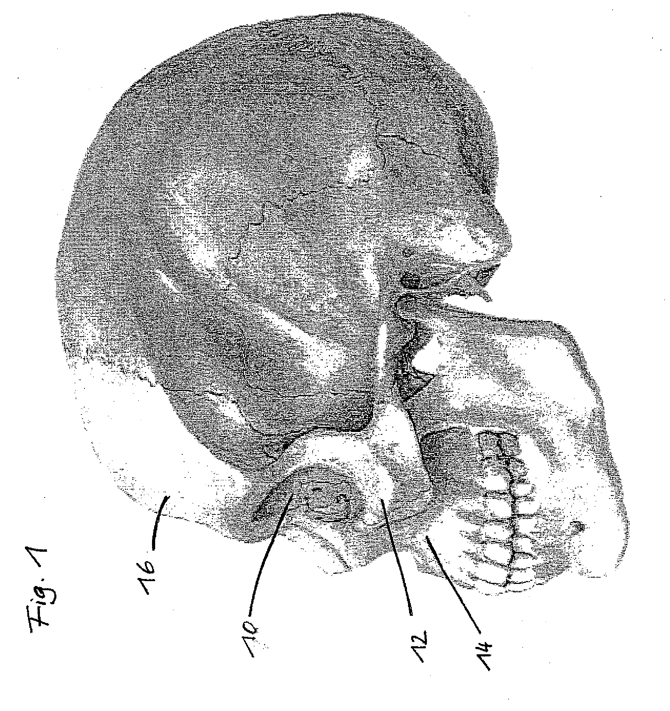

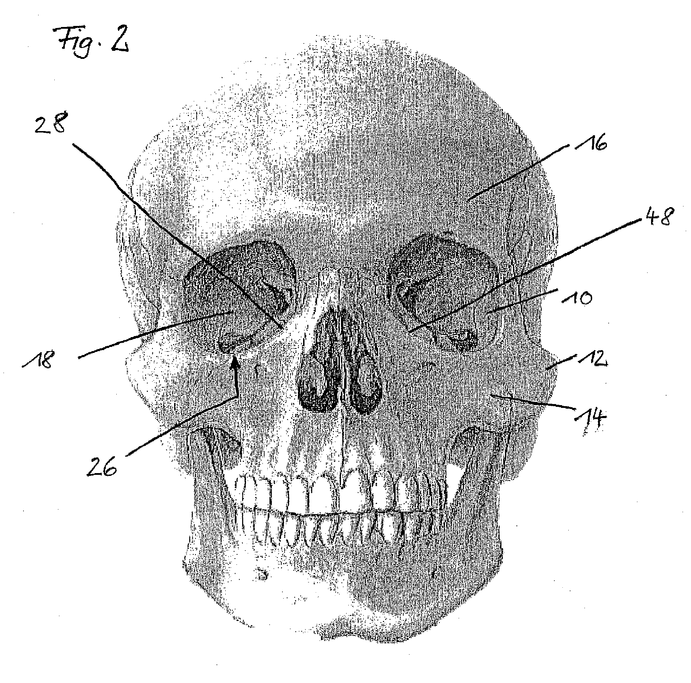

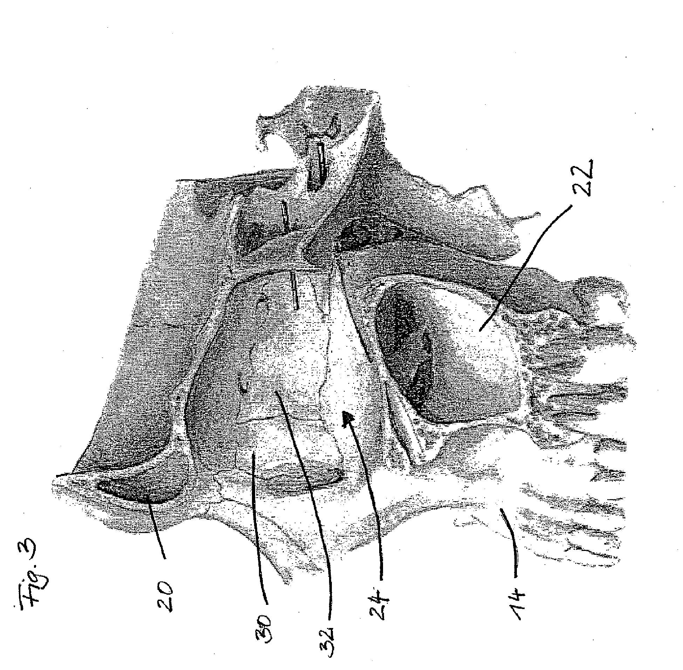

Wie es in den Fign. 1 bis 3, die mit freundlicher Erlaubnis der 3B Scientific GmbH, Hamburg (www.3bscientific.com) zur Verdeutlichung des Sachverhalts hierin verwendet werden, am Beispiel eines menschlichen Schädels gezeigt ist, bildet eine Augenhöhle (Orbita) 10 das schützende, knöcherne Gehäuse für Augapfel (Bulbus oculi) mit Sehnerv (Nervus opticus), weiteren Nerven und Blutgefäße sowie Tränendrüse (nicht gezeigt). Diese Strukturen sind von orbitalem Fettgewebe (Corpus adiposum orbitae) umgeben. Die Augenhöhle 10 hat die Form eines leicht nach außen und unten divergierenden Trichters, wobei die sechs Augenmuskeln an der Spitze des Trichters um den Sehnerv herum ihren Ursprung haben und am Augapfel ansetzen, der sich somit in der Augenhöhle wie in einer Gelenkhöhle bewegt. Der knöcherne Eingang der Augenhöhle 10 bildet einen stabilen Ring aus Jochbein (Os zygomaticum) 12, Oberkiefer (Os maxillare) 14 und Stirnbein (Os frontale) 16. Die übrige knöcherne Begrenzung besteht aus teilweise sehr dünnen Knochenlamellen 18. Diese Knochenlamellen 18 bilden auch die Grenze zu umliegenden Hohlräumen wie etwa der Stirnhöhle (Sinus frontalis) 20 oder der Kiefernhöhle (Sinus maxillaris) 22. Der Augenhöhlenboden (Paries inferior) 24 wird von zwei Knochen, dem Oberkiefer 14 und dem Jochbein 12 gebildet, die etwa in der Mitte des unteren Augenhöhleneingangs an der als Sutura zygomaticomaxillaris bezeichneten Schädelnaht 26 aneinander stoßen, wobei sich an den Oberkiefer 14 beim Übergang zur medialen Augenhöhlenwand 28 vorn das Tränenbein (Os lacrimale) 30 und weiter innen das Siebbein (Os ethmoidale) 32 anschließen.

Als Augenhöhlenbodenfraktur oder Blow-Out-Frakturen, die unter Mitwirkung des erfinderischen Instruments behoben werden soll, wird ein Bruch des Bodens 24 der Augenhöhle 10, hervorgerufen zum Beispiel durch eine direkte Gewalteinwirkung (z. B. durch einen Squash- oder Tennisball oder einen Faustschlag) oder im Zusammenhang mit einer komplexeren Fraktur etwa des Jochbeins 12, bezeichnet. Wird eine Augenhöhlenbodenfraktur (Orbitafraktur) zum Beispiel camputertomographisch diagnostiziert, wird ein operativer Eingriff zumeist als indiziert erachtet, denn häufig ist damit beispielsweise ein Austreten bzw. Einklemmen von Orbitainhalt (Herniation) in die Kiefernhöhle 22, ein Zurücksinken des Augapfels in die Augenhöhle (Enophthalmus) oder das Auftreten von Doppelbildern (Diplopie) verbunden. Eine operative Rekonstruktion der Augenhöhle ist jedoch nicht unumstritten, da sie unter Umständen hohe Risiken birgt. In wenigen Fällen - gemäß einer Studie an 20500 Patienten in 0,04 % aller Fälle - ist postoperativ sogar ein Visusverlust aufgetreten.

Bei einer infraorbitalen Zugang zur Behandlung einer Augenhöhlenbodenfraktur wird die Augenhöhle 10 chirurgisch gemäß der vorliegenden Erfindung von anterior eröffnet, wobei über einen Schnitt im Bereich des unteren Augenlides (palpebral) bis zum Rand der Augenhöhle 10 eingegangen, die Knochen- bzw. Beinhaut (Periost) eingeschnitten, um den Augenhöhlenunterrand darzustellen, und anschließend mit einem so genannten Freer das Weichgewebe (Augapfel, Augenmuskeln, Fettgewebekörper, Periost - alle nicht gezeigt) bis über die Fraktur vom Augenhöhlenboden 24 geschoben wird. Anschließend wird das Orbitaweichgewebe angehoben und eine sich auflösende Folie zur Stützung eingelegt.

Mit fortschreitender Dissektion muss jedoch immer mehr Weichgewebe weggehalten werden, was üblicherweise mit einem zunächst schmalen, dann breiteren Hirnspatel durchgeführt wird. Insbesondere dann, wenn auch ein Prolaps, d. h. ein Vorfall des den Augapfel umkleidenden Fettgewebes vorliegt, sind hohe Anforderungen an das Chirurgenteam gestellt, da in diesem Fall die Übersicht über das Operationsfeld erheblich beeinträchtigt sein kann.

Ein Hirnspatel ist jedoch kein optimales Instrumentarium für eine operative Versorgung von Augenhöhlenbodenfrakturen, da es - nomen est omen - nicht hierfür konzipert ist.

Es ist daher Aufgabe der vorliegenden Erfindung, ein Instrument zur Operation einer Augenhöhlenbodenfraktur, das eine operative Therapie erleichtert, einen Satz mit einer Mehrzahl solcher Instrumente und ein Verfahren zur Herstellung eines solchen Instruments bereitzustellen

Diese Aufgabe wird durch die Merkmale der Ansprüche 1, 6 bzw. 7 gelöst.

Gemäß der vorliegenden Erfindung umfasst ein Instrument zur Operation einer Augenhöhlenbodenfraktur einen löffelförmigen Endabschnitt, dessen Form sich an eine Form eines medialen Augenhöhlenbodens anschmiegt und einen an einem Ende mit dem löffelartigen Endabschnitt verbundenen Griffabschnitt.

Die Ausbildung dieses löffelförmigen Endabschnitts ist somit in Größe und Form an jene des Bereichs einer Augenhöhle 10 angepasst, an dem das Instrument erfindungsgemäß eingesetzt werden soll, wobei die Form durch Abformen des Augenhöhlenbodens eines Schädels ermittelt wurde. Bei der Operation wird das Instrument mit seinem löffelförmigen Endabschnitt von anterior im Wesentlichen im Bereich des Oberkiefers 14, teilweise übergreifend in den Bereich des Jochbeins 12 einerseits und in den Bereich von Tränenbein 30 und Siebbein 32 andererseits unter den wie oben beschrieben vom Augenhöhlenboden gelösten Orbitaweichteilen in die Augenhöhle 10 eingeführt, um den Augapfel mit seinen Anhanggebilden anzuheben und von der Fraktur abzuhalten. Wie es oben angedeutet ist, werden nach der Freipräparation der Fraktur etwaige in die Kieferhöhle hineinragende Orbitaweichteile aus dieser befreit, die Fraktur mit einer sich auflösenden Folie überdeckt und die Wunde anschließend verschlossen.

Die Form des erfindungsgemäßen Instruments wird hierin somit durch die komplizierte Form der Augenhöhle 10 bestimmt und definiert, da sie sich einer zufriedenstellenden begrifflichen Beschreibung entzieht. Durch die gegenüber herkömmlich verwendeten Hirnspateln, die eine über ihre Länge betrachtet mehr oder weniger die gleiche und vergleichsweise geringe Breite aufweisen, zumindest in einem in einer Längsrichtung mittleren Abschnitt des löffelförmigen Endabschnitts breitere und an die Anatomie des Augenhöhlenbodes 24 angepasste Form des erfindungsgemäßen Instruments übt der löffelförmige Endabschnitt durch das Anheben des Augapfels zwar größere Kräfte auf diesen aus, doch entspricht die Kraftverteilung, die auf die kaudale / kaudal-mediale Oberfläche des Augapfels wirkt, weitestgehend derjenigen, die durch die Knochenstruktur im normal-positionierten Auge ausgeübt wird. Insbesondere wird durch den Einsatz des erfindungsgemäßen Instruments der Augapfel insgesamt leicht angehoben und nicht lokal verformt bzw. eingedrückt, wie es bei herkömmlichen Hirnspateln aufgrund ihrer schlanken Form leicht der Fall ist, so dass die Verletzungsgefahr verringert ist. Insbesondere ist man bei herkömmlich verwendeten und insgesamt wesentlich schmaler ausgebildeten Hirnspateln, durch die der Augapfel sehr viel stärker eingedrückt wird, verleitet "nachzufassen", wodurch die Gefahr besteht, dass das Hirnspatel zu tief eingeführt und die Nervenstruktur (insbesondere der Sehnerv), der nach hinten austritt, verletzt wird. Der erste Abschnitt des erfindungsgemäßen Instruments verjüngt sich zwar zu seinem Ende hin, doch verbreitert er sich andererseits von diesem Ende weg deutlich, nimmt also diesbezüglich in etwa die Form eines Esslöffels ein, so dass ein im Vergleich zu dem Fall eines "paddelförmig" ausgebildeten herkömmlichen Hirnspatels tieferes Eindringen in die Augenhöhle 10 erschwert und die Gefahr einer Verletzung des Sehnervs verringert ist.

Obgleich die Erfindung vorzugsweise auf eine Anwendung in der Humanmedizin abzielt, ist eine Anwendung in der Veterinärmedizin, insbesondere bei verletzten Säugetieren vergleichbarer Anatomie durchaus denkbar.

Gemäß einer Ausgestaltung der vorliegenden Erfindung weist das Instrument einen zweiten Endabschnitt auf, der mit dem anderen Ende des Griffabschnitts verbunden und als ein sich von dem Griffabschnitt weg verjüngender Hirnspatel ausgebildet ist. Dies stellt eine praktische Verbindung von zwei unterschiedlichen Werkzeugen in einem manuellen Instrument dar, was ein konzentriertes und effizientes Operieren ermöglicht.

Gemäß einer weiteren Ausgestaltung der vorliegenden Erfindung ist der Griffabschnitt plastisch verformbar. Dies erlaubt eine für die Operation optimale Positionierung und/oder Einführung des jeweiligen Endabschnitts, und zwar derart, dass die Sicht des Chirurgen zu keinem Zeitpunkt der Operation durch seine Hand oder die eines assistierenden Arztes verdeckt ist. Das Instrument kann vorzugsweise am Übergang des Griffabschnitts zu dem als löffelartigen Endabschnitt ausgebildeten Arbeitsteil manuell verbogen werden, um eine optimale Wirkungsweise zu erzielen. Das Verhältnis der Länge des Griffabschnitts zu der des Arbeitsteils beträgt zum Beispiel 2:1.

Gemäß einer weiteren Ausgestaltung der vorliegenden Erfindung ist der löffelartige Endabschnitt asymmetrisch ausgebildet, was angesichts der asymmetrischen Form der Augenhöhle 10 vorteilhaft ist und aus der Herstellung des Instruments durch Abformung des Augenhöhlenbodens resultiert. Hierbei bedeutet asymmetrisch das Fehlen jeder Form von Symmetrieebene- oder Punkt. Diese vorteilhafte Ausbildung bringt es mit sich, dass es zwei Varianten des erfindungsgemäßen Instruments gibt: eine für das linke Auge, eine dazu spiegelbildliche für das rechte Auge. Außerdem ist eine Anpassung an das Alter des Patienten zweckmäßig, da sich nicht nur die Größe, sondern auch die Form der Augenhöhle im Laufe der Ontogenese, der Entwicklung des Individuums, verändern. Insbesondere ist eine solche Veränderung während der so genannten Synostose, d. h. der knöchernen Verwachsung der Schädelknochen an den zu den Syndesmosen zählenden Schädelnähten bzw. Fontanellen zu beobachten, die so charakteristisch ist, dass das Entwicklungsstadium dieser Verbindung neben dem Stand der Zahnentwicklung zur Altersbestimmung eines Menschen herangezogen wird. Dies ist erfindungsgemäß durch die Bereitstellung verschiedener Größen berücksichtigt, wobei die vorliegende Erfindung mindestens zwei Größen, für Erwachse und Kinder, vorsieht.

Gemäß einer weiteren Ausgestaltung der vorliegenden Erfindung ist das Instrument einteilig aus einem Metall, insbesondere einer medizinischen Legierung gebildet.

Gemäß einer weiteren Ausgestaltung der vorliegenden Erfindung enthält ein Instrumentensatz mit einer Mehrzahl von Instrumenten gemäß der vorliegenden Erfindung zwei Instrumente für das linke und das rechte Auge eines Erwachsenen und /oder zwei Instrumente für das linke und das rechte Auge eines Kindes, wobei sich, wie es oben beschrieben ist, die "Erwachsenen-Instrumente" in Größe und Form von den "Kinder-Instrumenten" unterscheiden. Die Erfindung ist jedoch nicht auf zwei Größen/Formen begrenzt. Darüber hinaus ist auch eine ethnologische Anpassung denkbar, da die Schädelformen der einzelnen Völker sehr verschieden sind.

Gemäß einer weiteren Ausgestaltung der vorliegenden Erfindung umfasst ein Verfahren zur Herstellung eines Instruments nach einem der Ansprüche 1 bis 5 die Schritte: Erstellen eines Abdrucks durch direkte Abformung eines Augenhöhlenbodens eines Schädels, und Fertigung des Instruments als Standardinstrument anhand des Abdrucks. Vorzugsweise wird der Abdruck als Wachsabdruck oder dergleichen entnommen, um ein anatomisch möglichst präzises Instrument zu erhalten. Anhand des Abdrucks wird ein Standardinstrument hergestellt. Vorzugsweise werden Standardinstrumente in zwei oder mehreren Größen angefertigt. Durch die dadurch erreichte Standardisierung kann es vermieden werden, für jeden einzelnen Patienten ein eigenes Instrument anzufertigen.

Die obigen und weitere Eigenschaften und Vorteile der vorliegenden Erfindung sind aus der nachfolgenden detaillierten Beschreibung, die unter Bezugnahme auf die beigefügten Zeichnungen gemacht wurde, deutlicher ersichtlich. In den Zeichnungen sind:
Fig. 1 eine Seitenansicht eines menschlichen Schädels;
Fig. 2 eine Vorderansicht eines menschlichen Schädels;
Fig. 3 einen Sagittalschnitt eines menschlichen Schädels;
Fign. 4A und 4B eine Draufsicht bzw. eine Seitenansicht eines Instruments gemäß einer ersten Ausführungsform der vorliegenden Erfindung für ein linkes Auge eines Erwachsenen;
Fig. 5 eine vergrößerte Ansicht des löffelförmigen Endabschnitts der Ausführungsform des Instruments von Fig. 4A und Fig. 4B mit eingezeichneten Schnittverläufen A-O;
Fign. 6A - 60 Schnittansichten entsprechend den Schnittverläufen A bis O in Fig. 5;
Fign. 7A und 7B eine Draufsicht bzw. eine Seitenansicht eines Instruments gemäß einer zweiten Ausführungsform der vorliegenden Erfindung für ein rechtes Auge eines Erwachsenen;
Fig. 8 eine vergrößerte Ansicht des löffelförmigen Endabschnitts der Ausführungsform des Instruments von Fig. 7A und Fig. 7B mit eingezeichneten Schnittverläufen A-O;
Fign. 9A - 90 Schnittansichten entsprechend den Schnittverläufen A bis O in Fig. 8;
Fign. 10A und 10B eine Draufsicht bzw. eine Seitenansicht eines Instruments gemäß einer dritten Ausführungsform der vorliegenden Erfindung für ein linkes Auge eines Kindes;
Fig. 11 eine vergrößerte Ansicht des löffelförmigen Endabschnitts der Ausführungsform des Instruments von Fig. 10A und Fig. 10B mit eingezeichneten Schnittverläufen A-L;
Fign. 12A - 12L Schnittansichten entsprechend den Schnittverläufen A bis L in Fig. 11;
Fign. 13A und 13B eine Draufsicht bzw. eine Seitenansicht eines Instruments gemäß einer vierten Ausführungsform der vorliegenden Erfindung für ein rechten Auge eines Kindes;
Fig. 14 eine vergrößerte Ansicht des löffelförmigen Endabschnitts der Ausführungsform des Instruments von Fig. 13A und Fig. 13B; und
Fign. 15 - 15L Schnittansichten entsprechend den Schnittverläufen A bis L in Fig. 14.

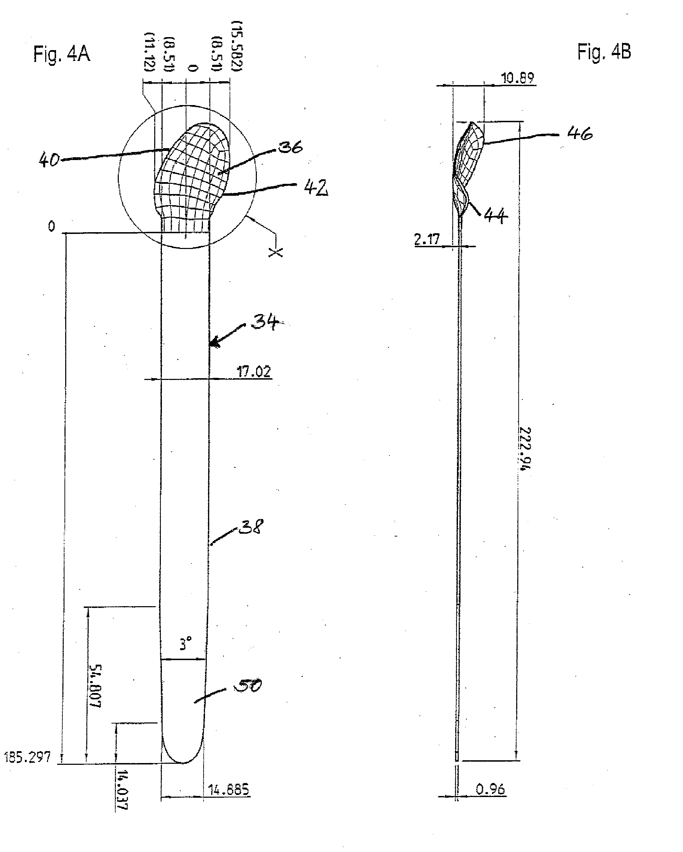

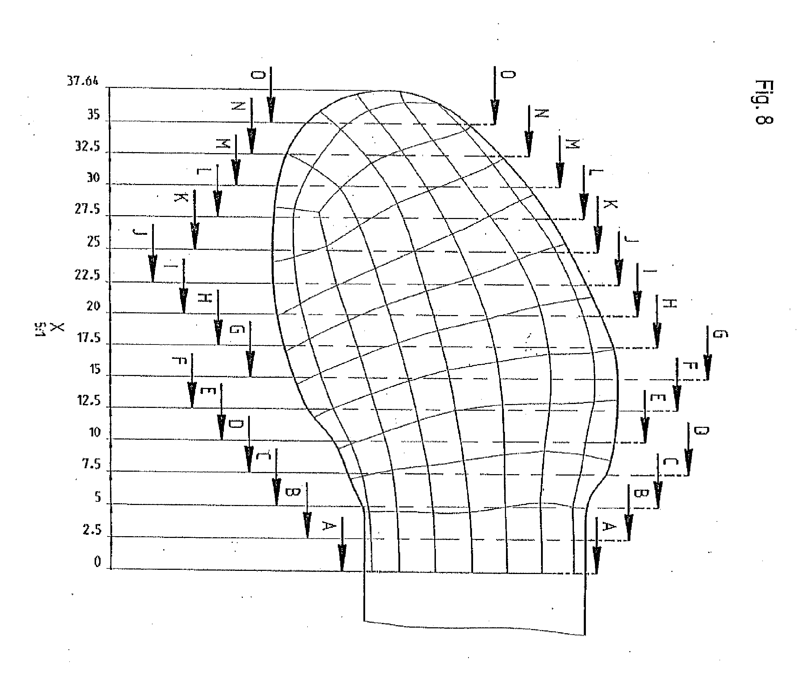

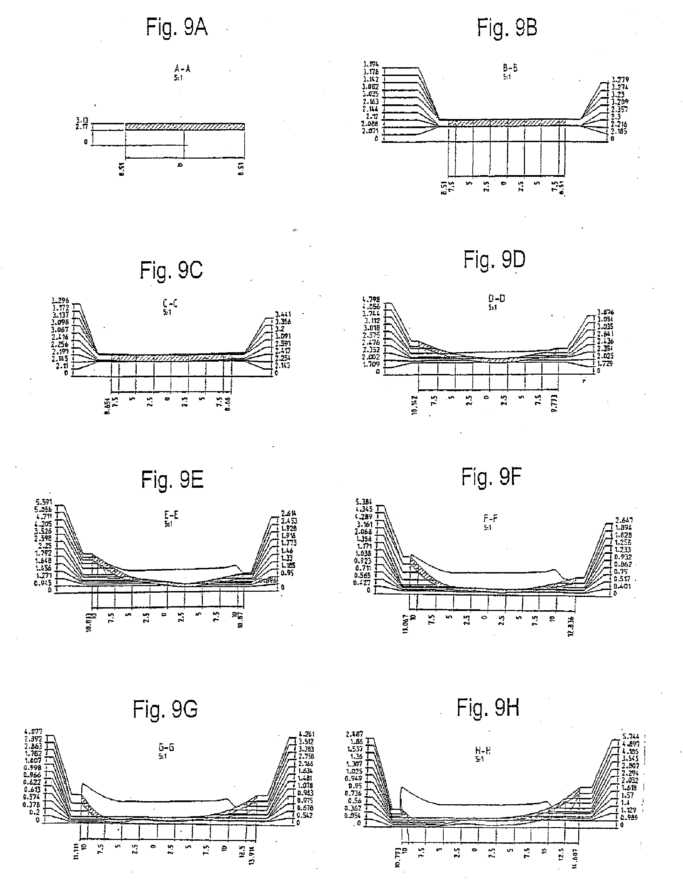

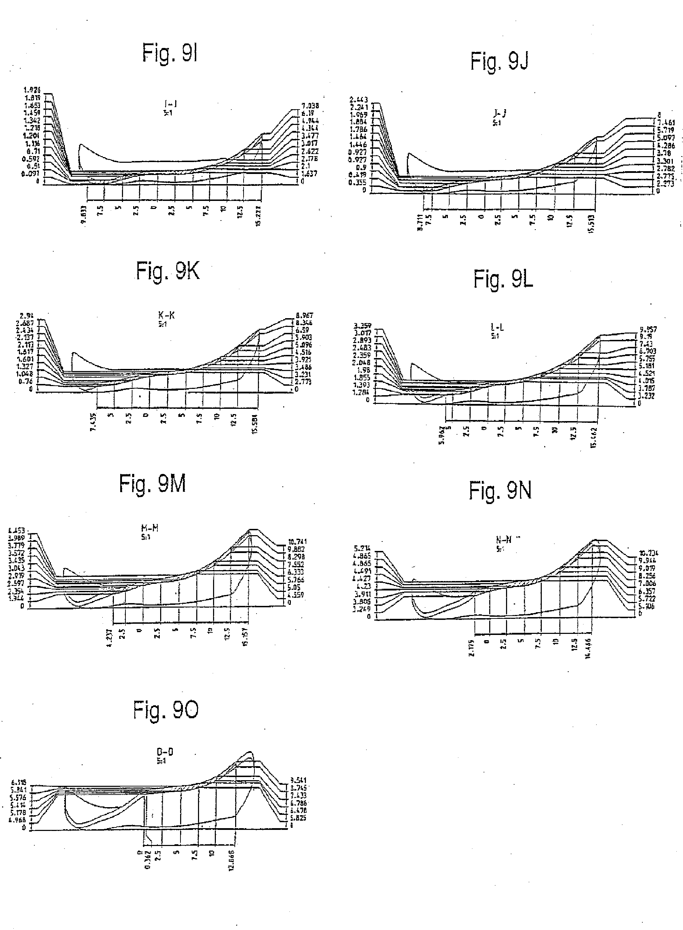

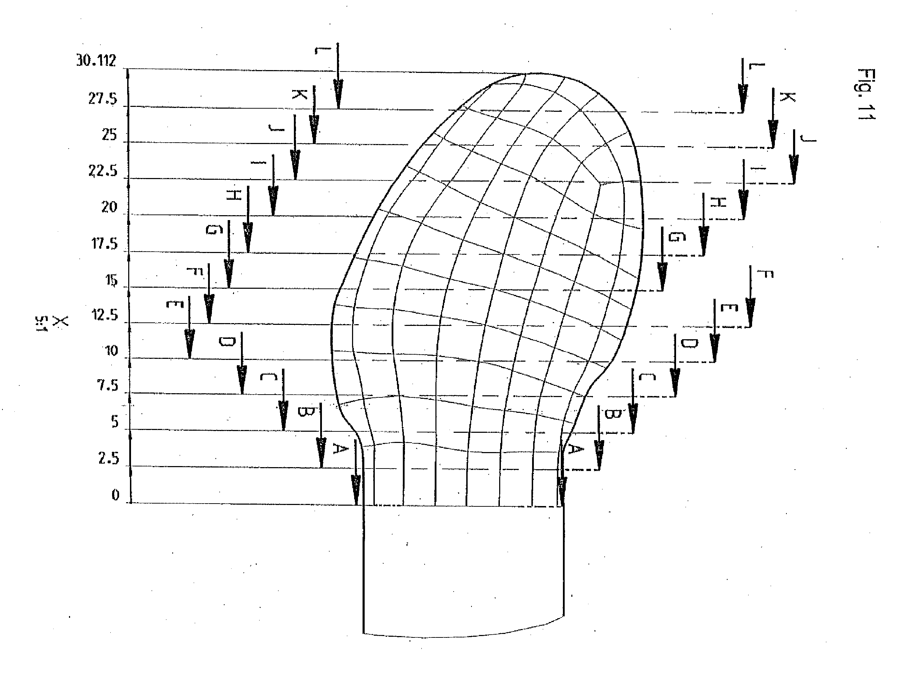

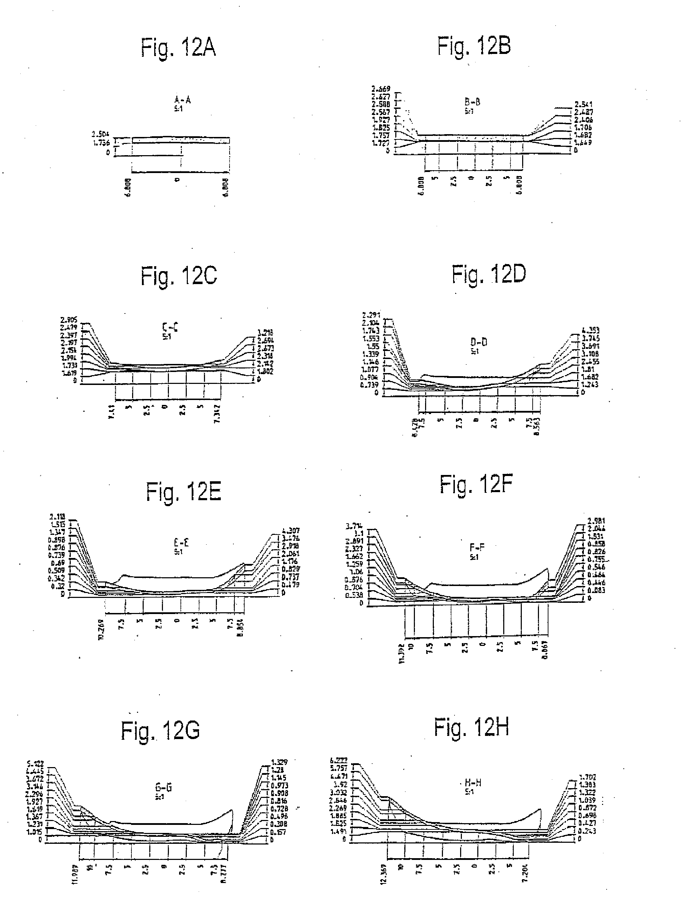

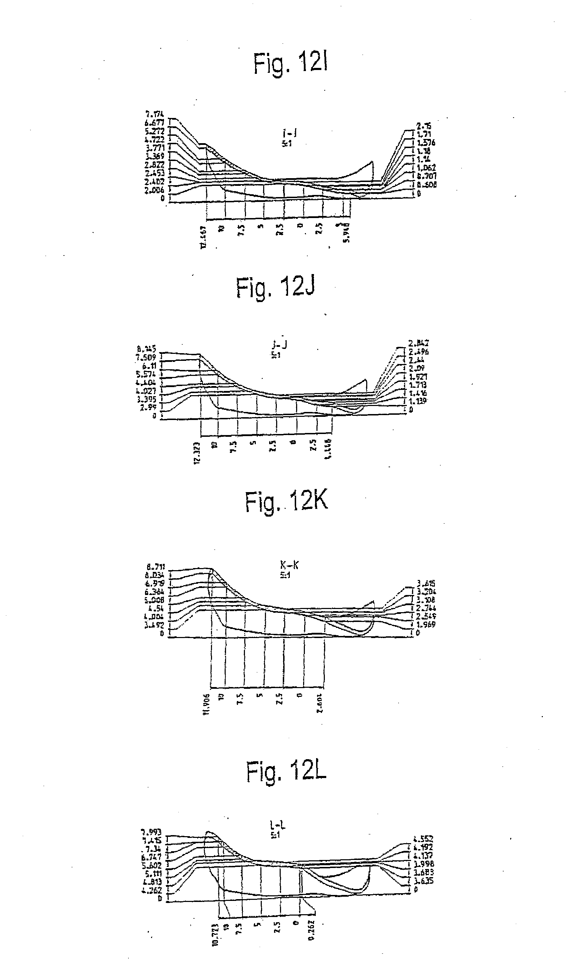

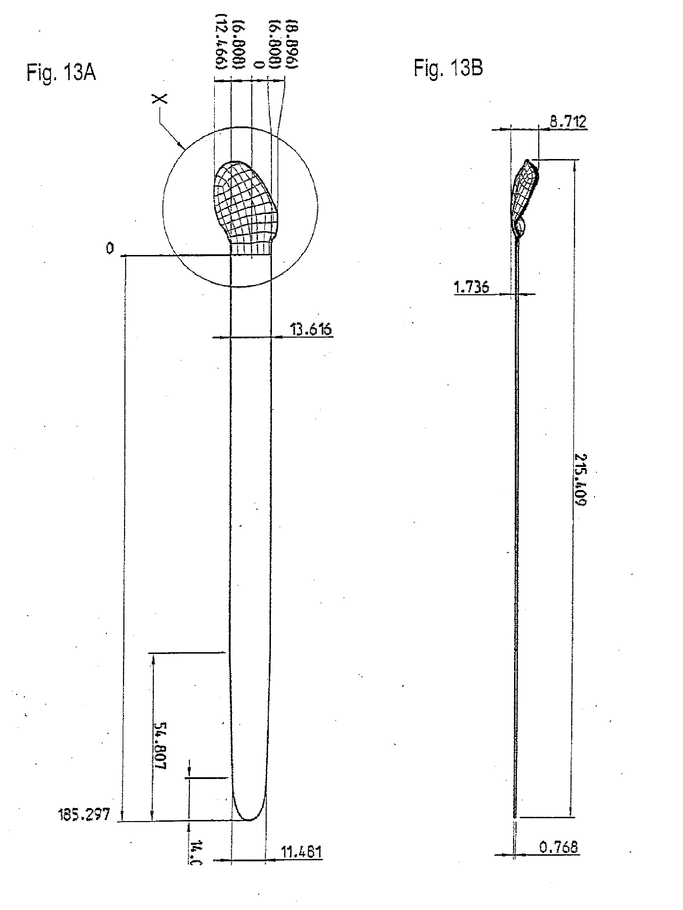

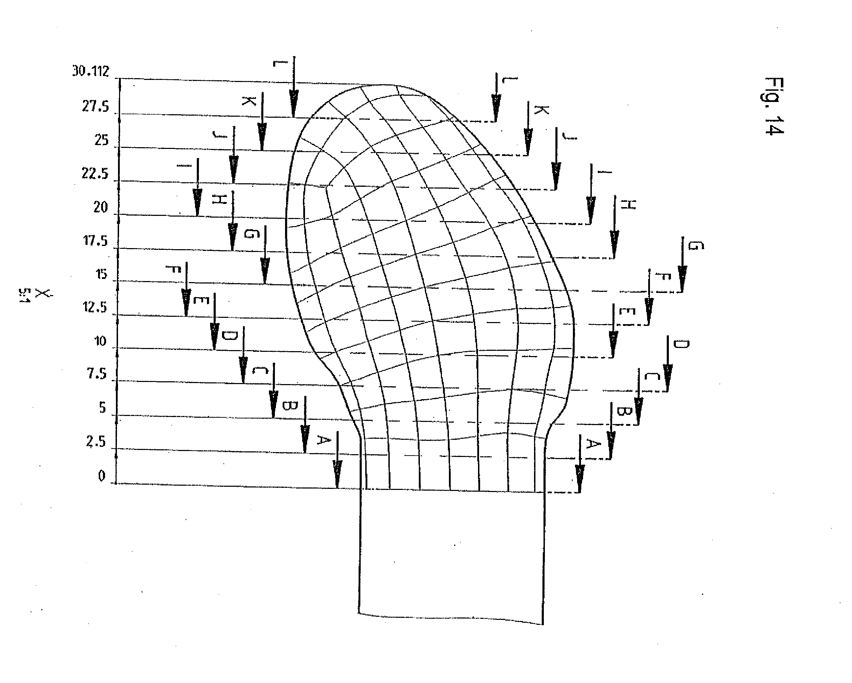

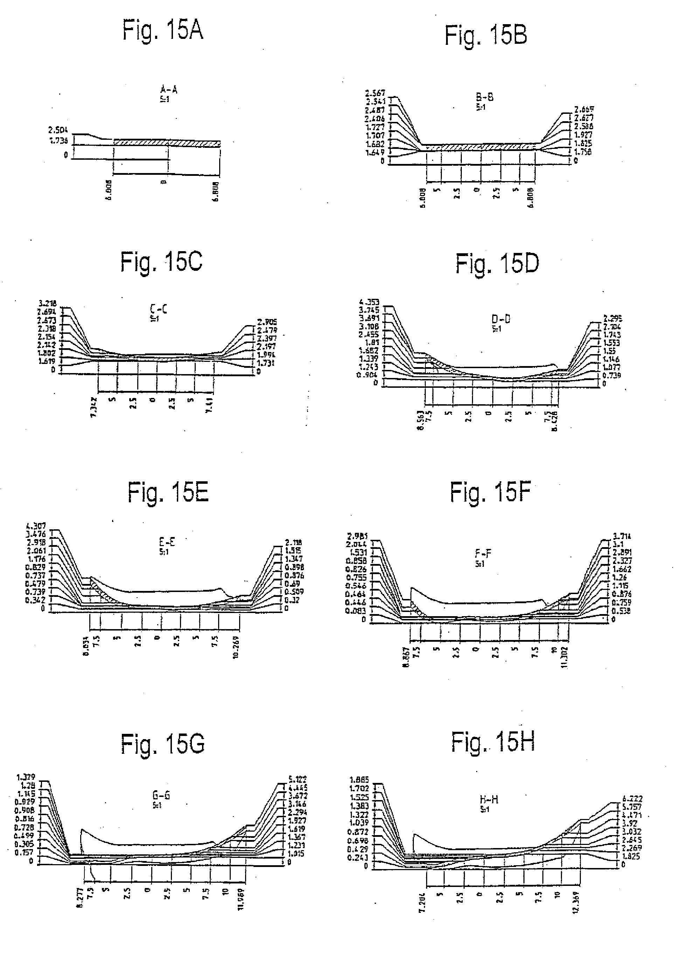

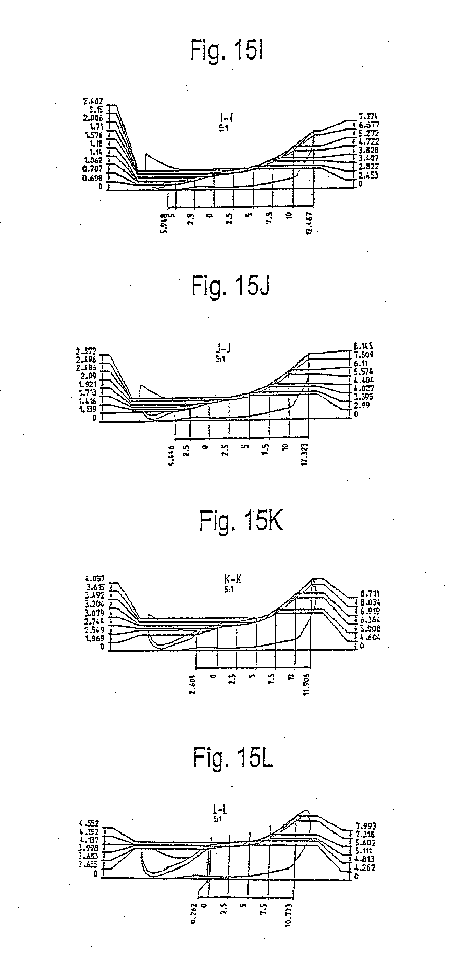

Die Fign. 4A bis 60 zeigen Ansichten eines Instruments zur Operation einer Augenhöhlenbodenfraktur gemäß einer ersten Ausführungsform der vorliegenden Erfindung, d. h. für ein linkes Auge eines Erwachsenen. Da die Form des erfindungsgemäßen Instruments bis auf die weiter unten genannten Unterschiede identisch ist, ist die nachfolgende Beschreibung auf diese Variante beschränkt.

Fign. 4A und 4B sind eine Seitenansicht bzw. eine Draufsicht eines Instruments 34 gemäß einer ersten Ausführungsform der vorliegenden Erfindung für ein linkes Auge eines Erwachsenen. In Fig. 4A ist deutlich die asymmetrische Form eines Endabschnitts 36 zu erkennen, der mit einem geradlinigen Griff 38 verbunden ist. Ein Seitenrand 40 ist so ausgebildet, dass er der Kontur der medialen Augenhöhlenwand angeglichen ist, während der gegenüberliegende Seitenrand 42 keiner Augenhöhlenwand nachgebildet ist; er befindet sich während der Operation im Bereich der Mitte des Augenhöhlenbodens. In Fig. 4B ist eine zum Betrachter hin gewandte erste Hochwölbung 44 und eine vom Betrachter abgewandte zweite Hochwölbung 46 zu erkennen. Die Hochwölbungen 44 und 46 entsprechen der anatomischen Form des Augenhöhlenbodens; ihre Form wurde experimentell durch direkte Abformung eines Augenhöhlenbodens an einem Schädel gewonnen und ist ideal zum Abhalten der Orbitaweichteile. Diese Form deckt den Bereich des Augenhöhlenbodens ab, der am häufigsten bricht, und dient ferner als Orientierungshilfe, um zu wissen, wie weit man gefahrlos in die Augenhöhle "hineinpräparieren" kann. Die in dem Endabschnitt 36 eingezeichneten Linien sollen dessen dreidimensionale Form visualisieren.

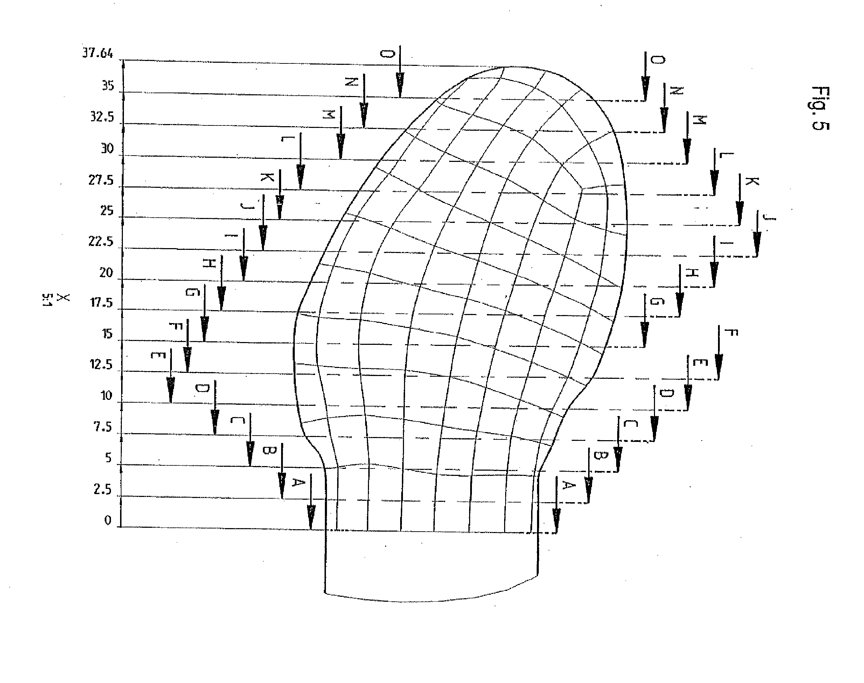

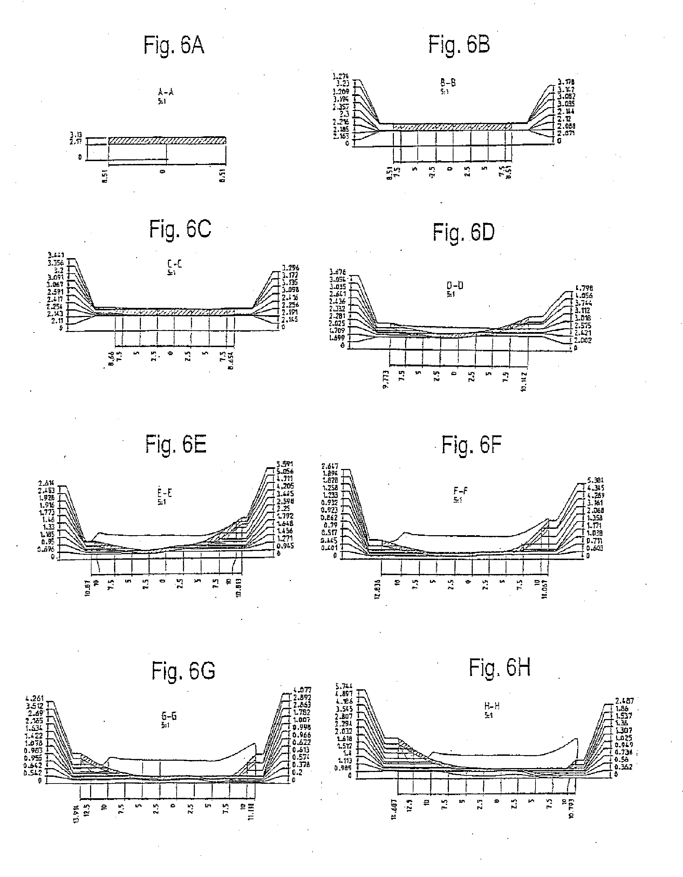

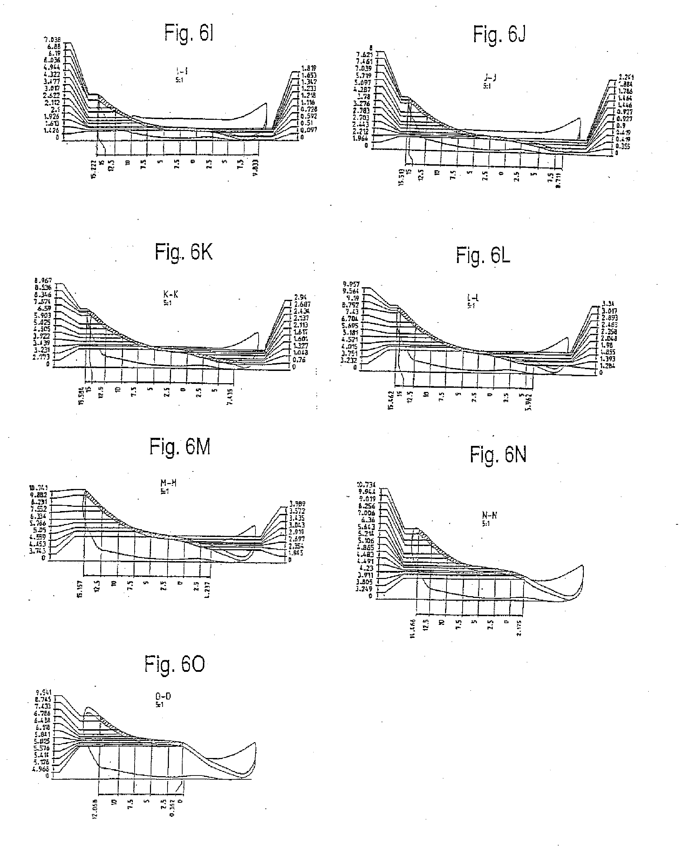

Die Fign. 6A - 60 zeigen Schnittansichten entsprechend den Schnittverläufen A bis O in Fig. 5 mit Blick in Richtung des Griffs 38. Insbesondere in den Fign. 6N und 60 ist klar die stark unterschiedliche Ausprägung der ersten Hochwölbung 20 (rechts) gegenüber der zweiten Hochwölbung 22 (links) zu erkennen.

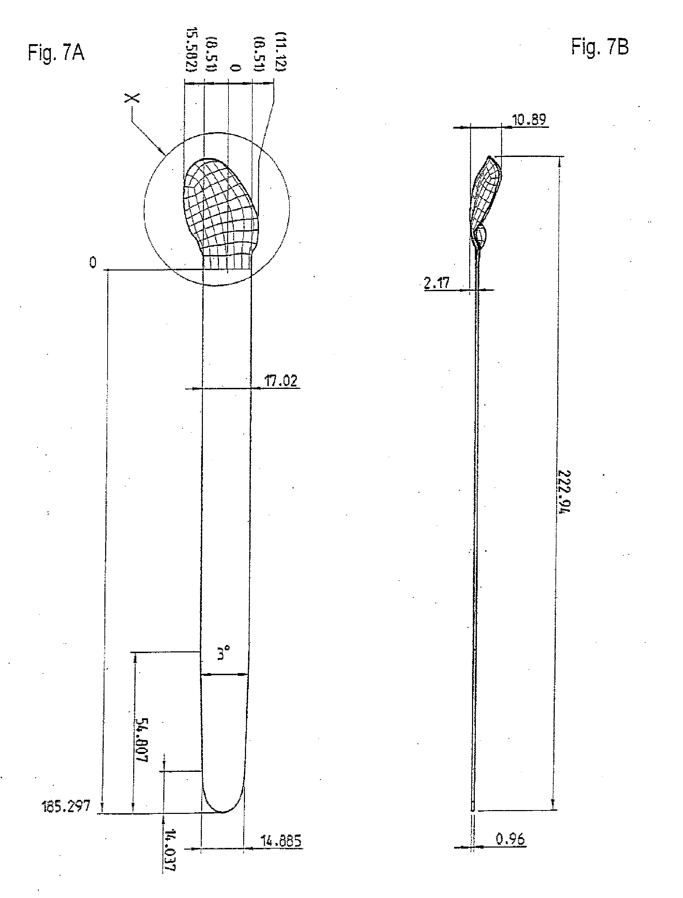

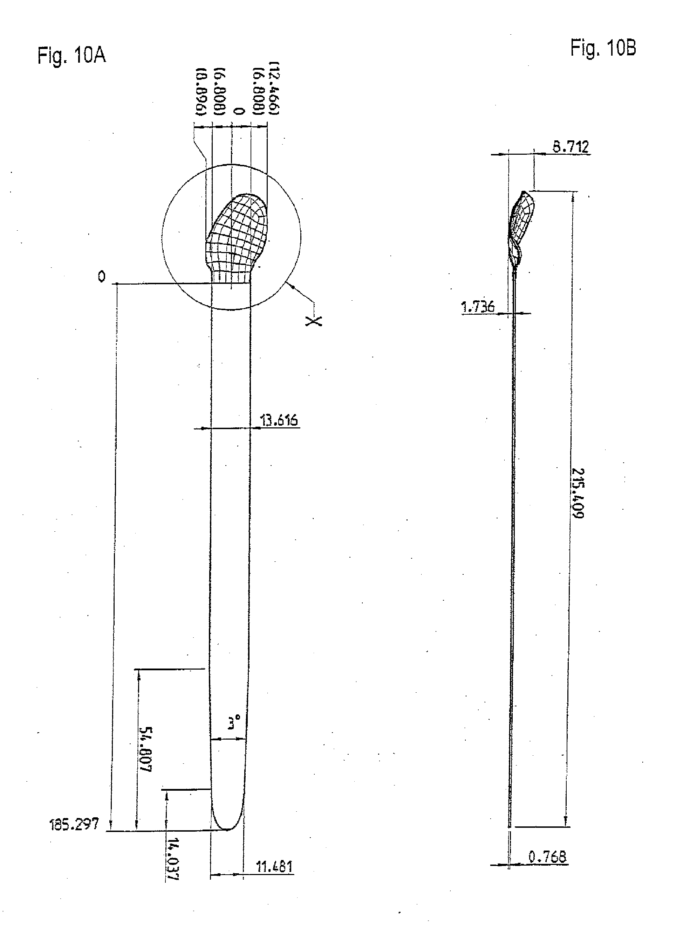

Ein Vergleich der Fign. 4A und 4B für ein linkes Auge mit den Fign. 7A bzw. 7B für ein rechtes Auge zeigt deutlich die Spiegelbildlichkeit des erfindungsgemäßen Instruments. Ferner zeigen die Maßangaben zum Beispiel in den Fign. 4A und 4B für einen Erwachsenen im Vergleich zu denen in den Fign. 10A bzw. 10B für ein Kind die Größenanpassung an das Alter des Patienten.

Wie es in den Fign. 4A und 4B gezeigt ist, weist das erfindungsgemäße Instrument 34 einen dem Endabschnitt 36 gegenüber liegenden zweiten Endabschnitt 48 auf, der in Form eines sich vom Griff 38 weg verjüngenden Hirnspatels ausgebildet ist.

Obgleich die vorliegende Erfindung bezüglich der bevorzugten Ausführungsformen offenbart worden ist, um ein besseres Verständnis von diesen zu ermöglichen, sollte wahrgenommen werden, dass die Erfindung auf verschiedene Weisen verwirklicht werden kann, ohne den Umfang der Erfindung zu verlassen. Deshalb sollte die Erfindung derart verstanden werden, dass sie alle möglichen Ausführungsformen und Ausgestaltungen zu den gezeigten Ausführungsformen beinhaltet, die realisiert werden können, ohne den Umfang der Erfindung zu verlassen, wie er in den beigefügten Ansprüchen dargelegt ist.

### Liste der Bezugszeichen

- 10: Augenhöhle
- 12: Jochbein
- 14: Oberkiefer
- 16: Stirnbein
- 18: Knochenlamellen
- 20: Stirnhöhle
- 22: Kiefernhöhle
- 24: Augenhöhlenboden
- 26: Schädelnaht
- 28: Augenhöhlenwand, medial
- 30: Tränenbein
- 32: Siebbein
- 34: Instrument gemäß der vorl. Erfindung
- 36: Löffelartiger Endabschnitt
- 38: Griffabschnitt
- 40: Seitenrand
- 42: Seitenrand, gegenüber 40
- 44: Hochwölbung (erste)
- 46: Hochwölbung (zweite)
- 48: Augenhöhlenrand
- 50: Zweiter Endabschnitt

## Patentansprüche

1. Instrument (34) zur Operation einer Augenhöhlenbodenfraktur, **gekennzeichnet durch** einen löffelförmigen Endabschnitt (36), dessen Form sich an eine Form eines medialen Augenhöhlenbodens anschmiegt, und einen an einem Ende mit dem löffelartigen Endabschnitt verbundenen Griffabschnitt (38).

2. Instrument (34) nach Anspruch 1, **gekennzeichnet durch** einen zweiten Endabschnitt (50), der mit dem anderen Ende des Griffabschnitts (38) verbunden und als ein sich von dem Griffabschnitt (38) weg verjüngender Hirnspatel ausgebildet ist.

3. Instrument (34) nach Anspruch 1 oder 2, **dadurch gekennzeichnet, dass** der Griffabschnitt (38) plastisch verformbar ist.

4. Instrument (34) nach einem der Ansprüche 1 bis 3, **dadurch gekennzeichnet, dass** der löffelartige Endabschnitt (36) asymmetrisch ausgebildet ist.

5. Instrument (34) nach einem der Ansprüche 1 bis 4, **dadurch gekennzeichnet, dass** es einteilig aus einem biegsamen Metall gebildet ist.

6. Instrumentensatz mit einer Mehrzahl von Instrumenten (34) nach einem der Ansprüche 1 bis 5, **dadurch gekennzeichnet, dass** er zwei Instrumente (34) für das linke und das rechte Auge eines Erwachsenen und / oder zwei Instrumente (34) für das linke und das rechte Auge eines Kindes enthält.

7. Verfahren zur Herstellung eines Instruments (34) nach einem der Ansprüche 1 bis 5, das die Schritte umfasst:
- Erstellen eines Abdrucks durch direkte Abformung eines Augenhöhlenbodens (24) eines Schädels;
- Fertigung des Instruments (34) als Standardinstrument anhand des Abdrucks.
